# EUROPEAN PATENT APPLICATION

(11) **EP 1 424 390 A1**
(43) Date of publication of application: **02.06.2004**
(21) Application number: 02754159.8
(22) Date of filing: 05.08.2002
(51) Int. Cl.: C12N 7/01

(54) **THE INTACT HEPATITIS C VIRUS AND THE METHOD FOR CULTURING IT IN A VITRO CELL CULTURE**

(30) Priority: 06.08.2001 CN 01124001
(71) Applicant: Yangling Daiying Biological Engineering Co., Ltd., Shanxi 712100 (CN)
(72) Inventor: TANG, Hengli 3/F, Establishment Building, Shanxi 712100 (CN); CHU, Yonglie 3/F, Establishment Building, Shanxi 712100 (CN); ZHANG, Shulin 3/F, Establishment Building, Shanxi 712100 (CN); GUO, Wenxia 3/F, Establishment Building, Shanxi 712100 (CN)
(74) Representative: Bublak, Wolfgang
(86) International application number: PCT/CN2002/000536
(87) International publication number: WO 2003/040356

(57) **Abstract**

The present invention relates to the establishment of an *in vitro* cell culture system for culturing the whole HCV proliferating virus by using molecular biology and gene recombinant technology. Said method comprises the step of amplifying from the serum of HCV patients the full-length HCV genome comprising the 98 nucleotides at the 3' end of the genome; site-specific mutating the NS5A and NS5B of HCV genome; inserting a marker gene IRES-GFP expression cassette into the NS5B 3' end in the mutated HCV genome; and transfecting the sensitive cells and culturing, to obtain the infectious HCV offspring virus.

## Description

### Field of Invention

The present invention relates to the use of molecular biology and gene recombinant technology to construct the complete hepatitis C virus (HCV) genome having suitable mutation, and through transfecting sensitive cells to establish an *in vitro* culture system for the whole hepatitis C virus.

### Background Technology

Human hepatitis C is an infectious disease caused by hepatitis C virus (HCV). After HCV infects those who are susceptible to HCV, HCV causes not only the acute infections but also the chronic infections, leading to liver fibrosis, hepatocirrhosis and even to hepatocellular carcinoma, and presenting a serious threat to human health. What is more important is that, owing to the lack of effective control and prevention measures, HCV infection is still prevailing over the world. In accordance with the incomplete statistics, about 170 million persons have been infected with HCV, and there has been an increase in millions of persons infected with HCV each year. In China, there has been near 2.5% of population (about 30 million people) infected with HCV, which belongs to the HCV highly-infected area. Accordingly, hepatitis C is a serious human disease with top priority given to its control and prevention as well as a hot point for concentrated studies.

HCV, hepatitis B virus (HBV) and AIDS virus (HIV) are three of the most dangerous blood infective viruses. Apart from hepatitis A virus (HAV) and hepatitis B virus (HBV), it has been realized since 1978 that there exists a new pathogenic factor to cause hepatitis, which can not be isolated and cultured successfully using the conventional lab method, nor can the existence of the new pathogen be identified. Until 1989, Choo et al used molecular biotechnology to clone the cDNA of the pathogen, which was classified into Flaviviridae family in accordance with its genomic structure and nucleic acid sequence, and later named as hepatitis C virus (HCV). The discovery of hepatitis C virus (HCV) is a huge success of modern molecular biotech in research on pathogen. However, HCV is the first human virus which has been confirmed neither by artificial isolation in culture nor by observing virus particles. At present, only the HCV genome can be directly obtained and used in analysis and research by the researchers, while the molecular biological property and many other characteristics of HCV are speculated based on the HCV genome.

The genome of HCV is a single stranded, positive-sense RNA molecule of about 9400 nucleotides in length. It has only one open reading frame (ORF) which encodes the precursor polypeptides with about 3000 amino acids. Also, there is a section of noncoding sequence (UTR or NTR) at each 5' and 3' ends of the genome. Since HCV genome is liable to variation, HCV can be divided into 6 genotypes and at least 30 sub-genotypes based on the gene sequence. After enzymatic digestion of the precursor functional protein encoded by the HCV genome, nearly 10 functional polypeptides are formed, including C, E1, E2/NS1, NS2, NS3, NS4A, NS4B, NS5A and NS5B, respectively. The 5' UTR region is related to the replication, transcription and regulation of the virus, whose sequence is highly conserved. The 3' UTR region is closely related to the virus replication and assembly, but it is worth pointing out that, at present, the 3' end of HCV genome sequence in GeneBank generally terminates at polyA. Accordingly, in the construction of cDNA in HCV genome, the researchers have not taken into account the latter 98 conservative nucleotide sequence. The applicant holds that this is one of the main causes why the whole HCV virus can not be successfully cultured.

HCV parasites upon the liver in the infected patients so that the virus content in the blood is low, so low is the amount of the virus antigen. The *in vitro* cell culture system for HCV has so far not been set up so that the complete HCV virus has so far not been obtained, whereby slowing down the research progress on HCV biological characteristics, immunological characteristics, pathogenicity, diagnostics, therapy and prevention. It becomes the bottleneck constraining the HCV research and the hot point and important project for which the scholars both home and abroad are seeking.

As far as the applicant knows, the establishment of HCV *in vitro* cell culture system in the past is, first of all, to select a sensitive passage cell strain or the primary hepotocyte culture as the cells for isolating HCV, and then the serum containing high titer of HCV from patients is used for direct isolation and culture, or the cloned HCV genome is used to transfect the cell for proliferating the HCV. Unfortunately, none of these methods can be successful and no proliferating HCV can be obtained. Although there are only a few reports (Dash et al 1997, Am J Pathology 151: 363; Yoo et al 1995, J Yirol 69: 32) indicating that HCV replication can be detected by using RT-PCR nested PCR after the HCV genome transfects human liver carcinoma strains Huh-7 or Hep G2, it can neither confirm the production of the infectious virus particles in cells. It also can not ensure that HCV exists for a long period and expresses in a high level in the transfected cells. Up to date, no stable and effective HCV *in vitro* passage cell culture system and experimental animal model have been reported. Also, no reports have so far been found concerning scientific research on *in vitro* cell culture of HVC complete virus, along with the related products on sale.

### Summary of Invention

The purpose of the present invention is to provide a hepatitis C virus having *in vitro* proliferation and infection activity. The present invention also provides an *in vitro* cell culture method for culturing the complete HCV particles (HCV virion). The present invention provides an indispensable pathogenic material for further studying the biological and immunological characteristics and pathogenicity of HCV as well as an effective *in vitro* cell experimental model for hepatitis HCV infection diagnosis, medicine screening and evaluation, and vaccine research.

HVC DY strain hepatitis C virus provided by the present invention has been deposited in the China General Microbiological Culture Collections Center with deposition number CGMCC No. 0588. HCV DY strain can be cultured in an *in vitro* cell culture. The virus titer in the supernatant without concentration can reach 10⁶ - 10⁷ genome copy/ml. The virus can be preserved in the refrigerator at -80°C for 6 - 8 months without any changes in its bioactivity, which can still infect human liver carcinoma cell (Huh-7) strain and pass on more than 5 generations *in vitro*.

The *in vitro* cell culture method of culturing the complete HCV infectious particles provided by this method comprises:
1) The whole genome of HCV including 98 nucleotides at the 3' end of the genome is amplified from the serum of HCV patients;
2) Site-specific mutation is carried out in NS5A and NS5B of HCV genome, mutating serine at 1979 in NS5A into isoleucine and arginine at 2884 in NS5B into glycine;
3) A marker gene IRES-GFP expression cassette is inserted after the NS5B 3' end in the mutated HCV genome; and
4) The sensitive cells are transfected with the full-length HCV genome containing site-specific mutation and *in vitro* cell culture are carried out to obtain infectious HCV offspring virus.

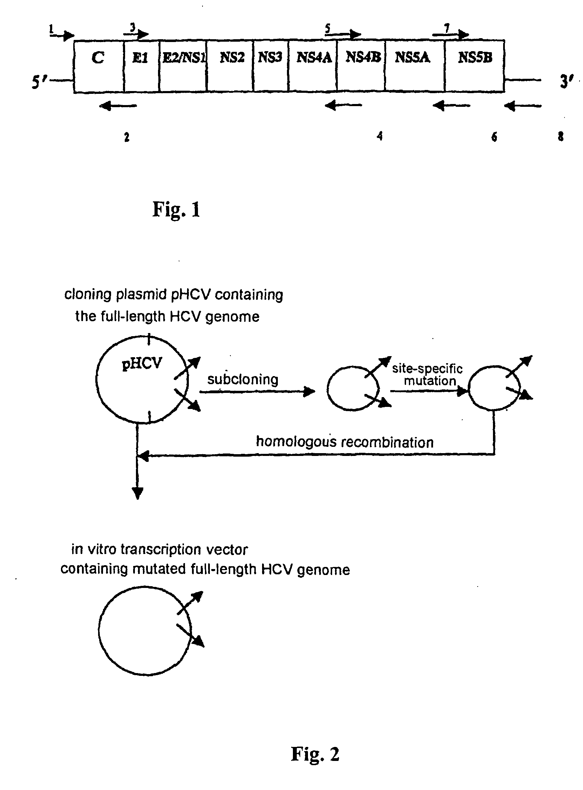

To begin with, serum specimen is extracted from the patients which are clinically confirmed to be suffered from hepatitis C. The HCV virus is extracted from the serum by ultracentrifugation. Then, the virus is condensed and resuspended in the small volume of DMEM without FBS. The standard RNA extraction method or commercial kit is used to extract RNA from the condensed virus particles. RNA precipitate is air-dried and dissolved with water having no RNAase. PCR primers are designed in accordance with HCV sequence. The full-length HCV genome sequences are amplified from the patient's blood. Since HCV genome is 9.6kb in length, it is difficult to complete the amplification via only one amplification. For this reason, we have designed several pairs of PCR primers (as shown in Fig. 1). The genomic cDNA is obtained via RT-PCR. Multiple PCR amplified fragments which cover the whole HCV genome is spliced together via restriction endonuclease site so as to form the full-length cDNA of HCV genome. Then, the full-length HCV cDNA is cloned into pSP72 under the control of high-efficient transcription promoter, so that the full-length HCV genome RNA can be easily transcribed *in vitro.* What should be particularly pointed out is that the present invention should guarantee the HCV genome transcription with high efficiency. For this reason, it should be particularly ensured that the 98 nucleotides at the 3' end of the genome is amplified so that it can be transcribed into HCV RNA *in vitro* with high efficiency.

In order to ensure that HCV is able to replicate, transcribe and synthesize protein. effectively in the *in vitro* cell culture system, site-specific mutation technology is used to create specific mutations in NS5A and NS5B of genomes. The mutated amino acid sites are serine at 1979 of NS5A region and arginine at 2884 of NS5B region. The method for designing mutation is indicated in Fig. 2. The recombinant plasmid DNA is *in vitro* amplified and digested by restriction endonuclease so as to clone the NS5A and NS5B gene in HCV genome into a small clone vector pUC 19. Then, Quickchang™ XL mutagenesis kit (Stratagene) is used to produce specific site mutation in the coding sequences of NS5A or NS5B. Finally, the mutated sequence is recombined into the full-length genome via homologous recombination.

In order to effectively screen and identify HCV replication and multiplication in cells, a selectable HCV genome is designed and constructed. The constructed HCV genome contains not only the artificially adapted mutated gene, but also an selectable marker gene. Green fluorescence protein (GFP) gene and an internal ribosome entry site (IRES) fusion sequence is added at the 3'end of the NS5B coding region. HCV replication is initially identified via screening GFP positive cells.

By the above method, a recombinant high-efficienct expression plasmid is constructed which comprises the full-length HCV genome, suitable site-specific mutation and selectable marker gene. The plasmid DNA is extracted by phenol/chloroform extraction and ethanol precipitation. The HCV genomic RNA is transcribed by using commercial *in vitro* high-efficient transcription kit (Ambion Company, MEGAscript™ kit, order No: 1334). Human hepato-carcinoma cell line Huh-7 has been found, through screening, to be the best cell line for supporting the replication of HCV. Said cells are cultured into monolayer. The HCV RNA is transferred into Huh-7 cells via gene gun and the cells are cultured. When the virus titer of HCV in the culture solution is determined to be up to 10⁶ - 10⁷ copy/ml, the virus is harvested and preserved in low temperature.

HCV proliferation in the *in vitro* cell culture system may be monitored or detected with the following methods:
(1) HCV PCR primers are designed for detecting HCV genome in the culture supernatant to prove the presence of nuclease-resistant HCV genomic RNA;
(2) The in-situ hybridization method with HCV RNA probes is employed to detect the positive and the negative strand of HCV RNA in the infected cells;
(3) The HCV protein polypeptide in the cell cultures is detected by immunofluorescence;
(4) The HCV titer and infectivity of the offspring virus may be determined.

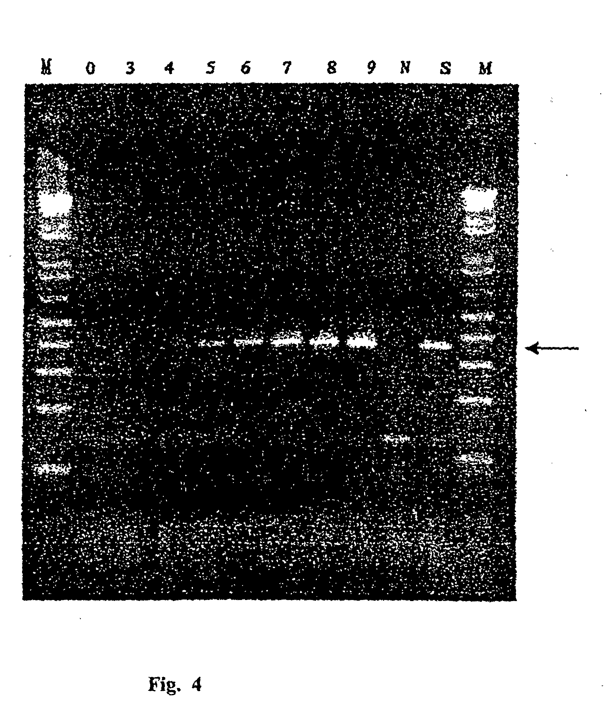

After detection, it has been demonstrated that the HCV genome constructed by the present invention can be replicated and proliferated in the culture system. The HCV titer in the supernatant can reach 1.9 x 10⁶ genome copy/ml (see Fig. 4) and said titer will not reduce after preservation at -80°C for 8 months. This HCV virus strain is named as DY strain (Daiying strain) and has been deposited in China General Microbiological Culture Collections Center with deposition number CGMCC No. 0588.

The whole HCV virus culture system of the present invention may be used in the research of biological and immunological characteristics of HCV, anti-HCV drug screening and identification, as well as the *in vitro* model of HCV genes therapy. The virus produced in this culture system can be used as an antigen for diagnosing HCV infection, can be used to prepare polyclonal or monoclonal antibodies, and can be used to prepare vaccine and be used in the identification of vaccine.

The present invention has opened up a new way for the in-vitro isolation of hepatitis C virus (HCV). It firstly establishes the HCV in-vitro cell culture system and successfully cultures the whole HCV virus. The HCV titer in culture solution can reach 10⁷ copy/ml. The virus can be preserved at -80°C for 8 months without losing activity. The present invention provides a resource of whole virus for basic research, drug development, vaccine research and preparation, and lays a better foundation for controlling HCV infection.

### Brief Description of Drawings

Fig. 1 schematically shows the HCV genome and the positions of multi-pairs of PCR primers.
Fig. 2 schematically shows the mutagenesis method for producing suitable mutation in the specific genes of HCV genome.
Fig. 3 shows the scheme for establishing the *in vitro* cell culture system for culturing the HCV complete virus.
Fig. 4 shows the electrophoresis results of quantifying the supernatant HCV titer by RT-PCR, wherein M indicates the nucleic acid molecular weight marker; numerals 0 - 9 indicate different concentrations of positive controls (10⁰ - 10⁹ genome copy/ml); N represents the negative control; S represents the supernatant with arrow indicating the HCV target segments of PCR amplification.
Fig. 5 shows the presence of HCV negative strand RNA intermediate in culture cells detected by using specific primers, i.e. the results of RT-PCR electrophoresis. In said Figure, M indicates the molecular weight marker; S is the RNA lysate of cell culture; N represents the negative control, with arrow indicating the amplified negative strand PCR products.
Fig. 6 shows the electrophoresis results of the supernatant HCV RNA detected by RT-PCR, wherein M indicates the molecular weight marker; 1 is the negative control; 2 is the culture supernatant, with arrow indicating the amplified HCV target segments.
Fig. 7 shows the results of in-situ hybridization by using HCV probes, indicating that there are a large amount of HCV RNA in the cytoplasm.
Fig. 8 is the photograph showing the expression results of HCV protein polypeptide in infected cells by immunofluorescence, wherein 8-1 and 8-3 are the phase contrast microscope photograph, while 8-2, 8-4 are the color photos corresponding to 8-1 and 8-3 indicating that there are a large amount of green fluorescence proteins in the infected cytoplasm.

### Specific Embodiments

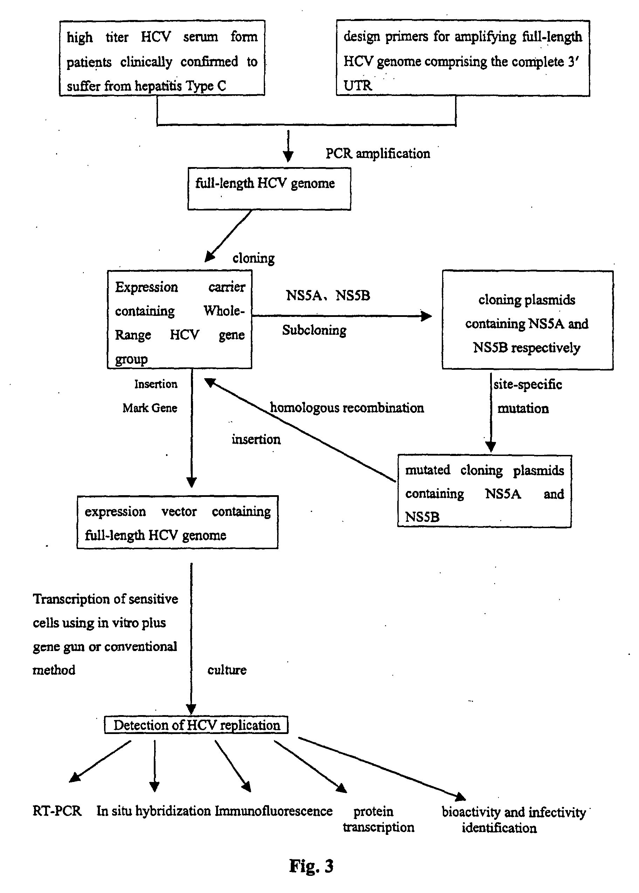

The scheme of this embodiment has been shown in Fig.3, and has been generally described as above.

Specifically, the process for amplifying the full-length HCV genome is, first, to design 8 PCT amplification primers based on the enzyme site (restriction site) on HCV conserved sequences, vector and specific segment of HCV genome. The full-length 9.6kb genome is gradually amplified by overlapping RT-PCR method. All the eight synthesized primers are provided with an enzyme-digested site for cloning and their positions ensure obtaining each segment of HCV genome so as to link them together to form the complete full-length HCV genome in terms of sequences, particularly with the nucleotide sequence at the 3' end of the genome.

The 8 primer sequences are as follows:

Primer 1:
GCCGAATTCGCCAGCCCCCTGATGGGGGC(SEQ ID NO: 1)
EcoR I

Primer 2:

CTCAGCCCGGGTACCCGGGCTG (SEQ ID NO: 2)

Kpn I

Primer 3:

CTCAGCCCGGGTACCCTTGGCCCCTC(SEQ ID NO: 3)

Kpn I

Primer 4:

CAAAAGAGTCTAGAATTACTATCTTG(SEQ ID NO: 4)

Xba I

Primer 5:

AAGATA GTAATTCTAGACTCTTTGAAC(SEQ ID NO: 5)

Xba I

Primer 6:

GCCAAGCTTAAA AAA AAA AAA GGG GGA TGG CCT ATT GGCC(SEQ ID NO: 6)

HindIII

Primer 7:

CGCCATCCC CCT TTT TTT TTT TTA AGCTTT TTT TTT TTT T(SEQ ID NO: 7)

Hind III

Primer 8:

GCC AAG CTT ACA TGA TCT GCA GAG AGG (SEQ ID NO: 8)

Hind III

The direction of the primers is from 5' to 3'. When PCR amplifications are carried out, primer 1 should be used with primer 2, primer 3 with primer 4, primer 5 with primer 6, and primer 7 with primer 8.

Total RNAs are extracted from sera of the patient suffering from hepatitis type C and used as the PCR amplification templates. Four amplification reactions are carried out by using conventional RT-PCR and the above synthesized 8 primers. Primer 1 and Primer 2 are used to get a 0.6kb HCV genome 5' end sequence segment. Primer 3 and primer 4 are used to get a 6.5Kb large segment of HCV genome. Primer 5 and primer 6 are used to get a 2Kb segment near the 3' end of HCV genome. Primer 7 and primer 8 are used to amplify the 0.4Kb micro-segments of HCV genome 3' terminal.

The HCV target segments obtained from RT-PCR (as shown in Fig. 1) are sequentially cloned and linked into high-efficient transcription vector pSP72, and finally the clone containing the full-length HCV genome is obtained. Specifically, the 0.6Kb PCR fragment is digested with EcoRI/KpnI and then cloned into the EcoR1/Kpn1 site in pSP72 to construct the recombinant plasmid pSP72-1. Then, the 6.5Kb large fragments of HCV genome is digested with KpnI/XbaI and cloned into KpnI/XbaI site of pSP72-1 which links after the above 0.6kb fragment to construct the recombinant plasmid pSP72-2. The third 2Kb fragment is digested by XbaI/HindIII, cloned into the XbaI/HindIII sites of pSP72-2 and put behind the 0.6kb-6.5kb fragments to construct recombinant plasmid pSP72-3. The 0.4Kb fragment of 3 terminal HCV genome (the 4^{th} PRC product) is digested by HindIII and cloned into the HindIII site of pSP72-3 which is behind the HCV 2 Kb fragment, so as to obtain the recombinant plasmid pSP72-HCV. In this way, the full-length HCV genome is obtained by multiple overlapping RT-PCR and step-by-step cloning and is successfully cloned between EcoRI site and HindIII site in pSP72 plasmid DNA. The results of restriction endonuclease analysis, PCR amplification and DNA sequence analysis all demonstrate that the full-length HCV genome has been obtained.

Site-specific mutagenesis is used to produce the mutation in specific sequence site of the cloned HCV genome so as to favor the effective biochemical synthesis and culture of HCV in cell culture. The scheme is shown in Fig. 2. Specifically, NS5A and NS5B of HCV genome are selected as the target genes for operation. First, DNA of pSP72-HCV is prepared. NS5A and NS5B genes are isolated from the HCV genome sequence and cloned into vector pUC19, respectively. Then, Quickchang™ XL mutagenesis kit (Stratagene) is used to mutate the serine at 1979 in NS5A region into isoleucine and the arginine at 2884 in NS5B region into glycine, whereby making the coding sequences of NS5A, NS5B genes in HCV genome have suitable and site-specific mutations. The mutated HCV sequence is recombined into the full-length HCV genome via DNA homologous recombination. Thus, the full-length HCV genome with suitable mutations in specific sequence is obtained.

A sufficient amount of expression plasmid DNA comprising the full-length HCV genome is prepared with high purity. Then, MEGAscript™ *in vitro* transcription kit (Ambion Company, US) is used to obtain a great bulk of HCV genomic RNA and the HCV genomic RNA is transfected into human liver carcinoma cell strain Huh-7 by gene gun. The cell is cultured for examining its supernatant. When there is high titer of HCV in the supernatant, the supernatant is harvested and preserved at -80°C. The Huh-7 cell may also be transfected with the recombinant plasmid DNA by conventional method to obtain HCV offspring virus.

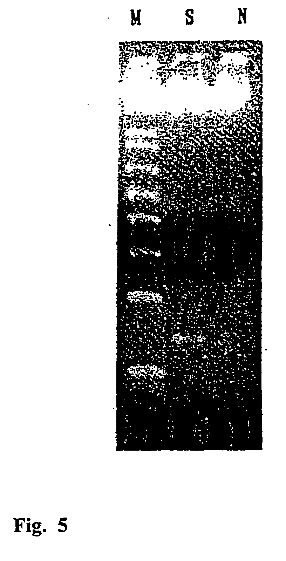

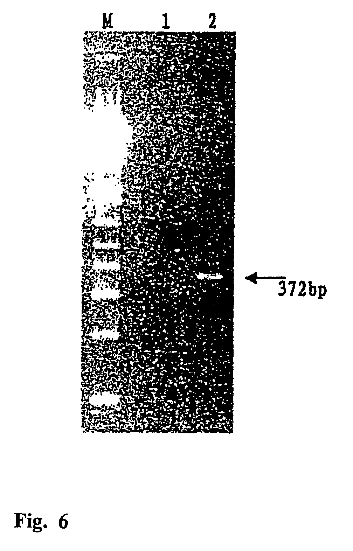

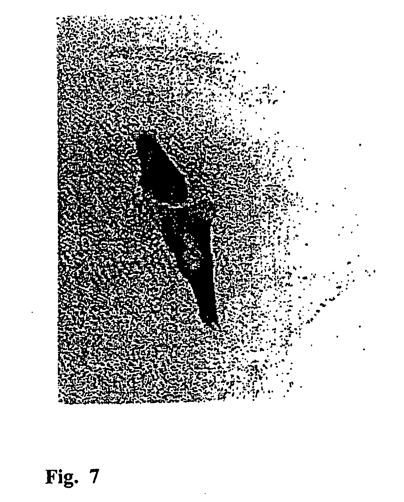

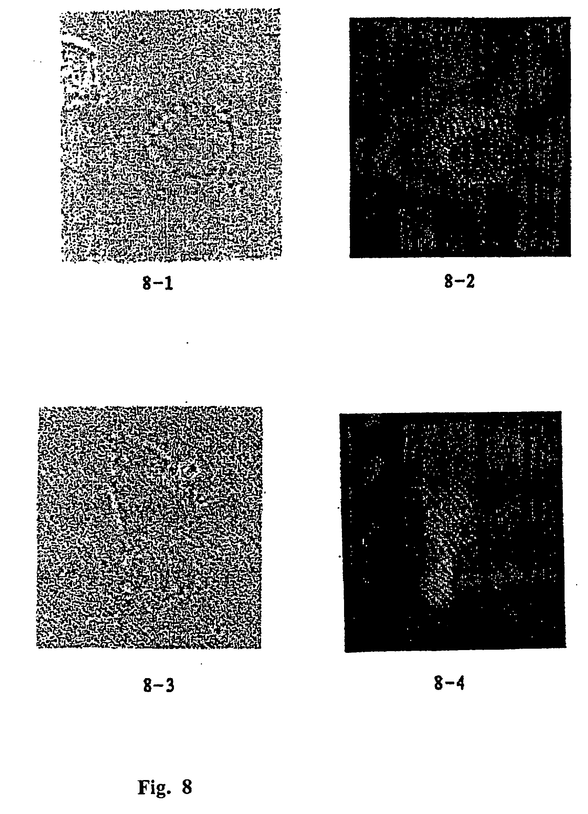

The following systematic identifications have been made on the HCV virus obtained by using the above methods:
(1) RT-PCR is used to qualitatively examine whether RNA of HCV exists in the supernatant (see Fig. 6);
(2) RT-PCR specific primers are designed and used to amplify the negative strand RNA which is the intermediate in the HCV replication, for confirming the HCV replication in the cells (see Fig. 5);
(3) The in-situ hybridization method with HCV genomic RNA probes is employed to demonstrate the existence of a large amount of HCV genome in the infected cells (see Fig. 7);
(4) The infected cells are detected by immunofluorescence with NS5B antibody labeled with fluorescein. The infected cells displaying the specific fluorescence (see Fig. 8) indicate that there are HCV virus proteins synthesis in the infected cells;
(5) The obtained first generation of HCV virus (supernatant) is assayed by quantitative RT-PCR identification, indicating that HCV titer in the uncondensed supernatant is up to 10⁶ genome copy/ml.
(6) The first generation of HCV virus is successively passed on Huh-7 cells. The supernatant virus titer of the 4^{th} generation is still up to 10⁶ genome copy/ml.

In summary, qualitative and quantitative identifications have been made with respect to the HCV offspring in the supernatant from different aspects, such as the amplification of the HCV genome, the presence of the replication intermediate, the expression of the virus protein, and the biological activity and infectivity of the offspring virus.

## Claims

1. A complete Hepatitis C Virus (HCV) strain, wherein the genotype of said virus is HCV 1b and said virus is deposited at CGMCC with deposition number of CGMCC. 0588.

2. A method for *in vitro* cell culturing the complete HCV of claim 1, comprising :
a) amplifying from the serum of HCV patients the full-length HCV genome comprising the 98 nucleotides at the 3' end of the genome;
b) site-specifically mutating the NS5A and NS5B of HCV genome by mutating the serine at 1979 in NS5A region into isoleucine and the arginine at 2884 in NSB5 region into glycine;
c) inserting a marker gene IRES-GFP expression cassette into the NS5B 3' end in the mutated HCV genome; and
d) transfecting sensitive cells with the full-length HCV genome containing the site-specific mutation, and culturing said cell *in vitro* to obtain infectious HCV offspring virus.

3. The use of the HCV virus of claim 1, wherein said virus is used as an antigen in the research of HCV biological and immunological characteristics, HCV infection diagnosis, preparation of polyclonal antibody and monoclonal antibody, HCV gene therapy, model of anti-HCV drug screening, vaccine preparation and vaccine detection.


SEQUENCE LISTING

<110> YANGLING DAIYING BIOLOGICAL ENGINEERING CO., LTD

<120> THE INTACT HEPATITIS C VIRUS AND THE METHOD FOR CULTURING IT IN
A VITRO CELL

<130> 024133

<150> CN01124001.6
<151> 2001-08-06

<160> 8

<170> PatentIn version 3.1

<210> 1
<211> 29
<212> DNA
<213> artificial sequence

<220>
<221> misc_feature
<223> primer

<400> 1
gccgaattcg ccagccccct gatgggggc 29

<210> 2
<211> 21
<212> DNA
<213> artificial sequence

<220>
<221> misc_feature
<223> primer

<400> 2
gggccaaggg tacccgggct g 21

<210> 3
<211> 26
<212> DNA
<213> artificial sequence

<220>
<221> misc_feature

<223> primer

<400> 3
ctcagcccgg gtacccttgg cccctc 26

<210> 4
<211> 26
<212> DNA
<213> artificial sequence

<220>
<221> misc_feature
<223> primer

<400> 4
caaaagagtc tagaattact atcttg 26

<210> 5
<211> 27
<212> DNA
<213> artificial sequence

<220>
<221> misc_feature
<223> primer

<400> 5
aagatagtaa ttctagactc tttgaac 27

<210> 6
<211> 40
<212> DNA
<213> artificial sequence

<220>
<221> misc_feature
<223> primer

<400> 6
gccaagctta aaaaaaaaaa agggggatgg cctattggcc 40

<210> 7
<211> 40
<212> DNA
<213> artificial sequence

<220>
<221> misc_feature

<223> primer

<400> 7
cgccatcccc cttttttttt tttaagcttt tttttttttt 40

<210> 8
<211> 27
<212> DNA
<213> artificial sequence

<220>
<221> misc_feature
<223> primer

<400> 8
gccaagctta catgatctgc agagagg 27